# EUROPEAN PATENT APPLICATION

(11) **EP 4 385 485 A1**
(43) Date of publication of application: **19.06.2024**
(21) Application number: 22214112.9
(22) Date of filing: 16.12.2022
(51) Int. Cl.: A61K 8/23, A61K 8/25, A61K 8/31, A61K 8/73, A61Q 5/08

(54) **BLEACH POWDER AND METHOD FOR BLEACHING HAIR**

(71) Applicant: Wella Germany GmbH, 64295 Darmstadt (DE)
(72) Inventor: ALAM, Ehsanul, 64295 Darmstadt (DE); HERRLEIN, Mathias, 64295 Darmstadt (DE); RICHTER, Marcus, 64295 Darmstadt (DE); PRYADILOVA, Olga, 64295 Darmstadt (DE)
(74) Representative: Bandpay & Greuter

(57) **Abstract**

The presently claimed invention is directed to a bleach powder comprising at least one saturated acyclic terpane which is a saturated acyclic terpane having 10 to 40 carbon atoms, at least one persulfate salt, and at least one silicate selected from the group consisting of alkali metal silicates, alkali metal metasilicates and ammonium silicate.

## Description

### Technical Field

The presently claimed invention is directed to a bleach powder comprising at least one saturated acyclic terpane which is a saturated acyclic terpane having 10 to 40 carbon atoms, at least one persulfate salt, and at least one silicate selected from the group consisting of alkali metal silicates, alkali metal metasilicates and ammonium silicate.

### Background

Hair bleaching is a well-established technique in the hair cosmetic industry. Hair bleaching involves the application of an oxidizing agent to the hair for a period that is effective to achieve a desired lighter hair shade. Usually, hydrogen peroxide solution in concentrations ranging from 6 to 12 % by weight is the oxidizing agent. The hydrogen peroxide is applied to the hair under alkaline pH conditions and gradually lightens the shade of the hair by oxidizing the melanin that imparts the colour to the hair.

To enhance the lightening efficacy of the peroxide solution, a persulfate salt-based formulation can be incorporated as a "booster". The sodium, potassium or ammonium persulfate salt is provided in the form of a powder which is mixed with the hydrogen peroxide solution prior to use. The mixed product is then applied to the hair for a period of time that is effective to achieve a desired lighter hair shade.

Currently, hair bleaches are most commonly found in the form of a two-component kit. One component comprises the aqueous hydrogen peroxide composition and the second component comprises a powdered bleach composition that contains persulfate salts and alkaline agents which act as accelerators of the bleaching process, when the two components are combined. This powdered bleach composition may be used alone to bleach hair or in combination with an aqueous hydrogen peroxide composition. The hydrogen peroxide and persulfates are very reactive when combined and form nascent oxygen in addition to hydrogen and sodium sulfide. The nascent oxygen greatly facilitates oxidizing and bleaching of melanin from the hair.

The salts and other active ingredients are routinely provided in the form of a powdery mixture of ingredients which are then transformed into a paste by mixing with water or other liquids. The powdery mixture often has the disadvantage of dusting. The oxidizing and alkaline agents in the powdery mixture may be harmful, if ingested or inhaled, in particular hairdressers can develop respiratory symptoms by conducting a high number of bleaching sessions which are attributed to the dusting of bleach powders; therefore, it is desirable to obtain a hair bleach that does not cause this dusting.

De-dusted formulations have been developed to control or reduce the dusting in powdered bleaching mediums. The de-dusted formulation generally mixes the powder with at least one base oil, such as mineral oil, resulting in a cream-type formulation. These systems can be unstable due to separation of the oil and the salt phases or too waxy and, therefore, cosmetically un-appealing after premature formula separation. The separation also has the effect that different portions of the same batch of powder taken from different locations in the batch have different chemical compositions and, thus, provide a different bleaching effect.

US 7,803,355 B2 discloses an anhydrous paste comprising at least one peroxygenated salt and at least one polydecene.

US 9,149,660 B2 describes hair bleach compositions comprising a persulfate salt and an oil gel containing mineral oil, ethylene/propylene/styrene copolymer, and butylene/ethylene/styrene copolymer.

Hydrogen peroxide and persulfates are very reactive when combined and form nascent oxygen. The high reactivity increases the effectiveness of bleaching mediums that are obtained by combining hydrogen peroxide and persulfate salts. However, the increased effectiveness can result in higher damage to the hair fibres. The hair fibres can potentially be weakened and my become so brittle that breakage occurs during the consumers' normal hair care routine.

US 2007/0169285 A1 describes that the presence of an oil phase can reduce the damage of hair that occurs during bleaching.

Another particularly critical performance area for the customer is the provision of the desired resultant colour and also the effective coverage of grey hair. Specifically, while the amount of grey hair to be coloured varies considerably from consumer to consumer, the resultant overall appearance of the coloured hair demanded by the customer should be nearly identical for the naturally pigmented hair and the grey hair on head, with the added requirement that the initial coverage is maintained during the post dyeing washing and drying circle.

Hence, there is a need for providing stylists and customers with a powdery bleach composition that does not dust or only dust to a very small extent and can be formulated into a hair bleaching medium which is stable and leads to consistent and effective colouring of hair and covering of grey hair while reducing the level of damage to the hair.

Hence, it is an object of the presently claimed invention to provide a bleach powder composition that does not dust or only dust to a very small extent and can be formulated into a hair bleaching medium which is stable and leads to consistent and effective colouring of hair and covering of grey hair while reducing the level of damage to the hair.

### Summary

Surprisingly, it was found that the addition of a saturated acyclic terpane having 10 to 40 carbon atoms to a bleach powder composition comprising at least one persulfate salt and at least one basic silicate prevents dusting of a bleach powder composition, as observed by hair stylists, which can be effectively formulated into a hair bleaching medium that leads to effective colouring of hair and covering of grey hair while reducing the level of damage to the hair, as evidenced by a lower combing force.

Accordingly, in one aspect, the presently claimed invention is directed to a bleach powder comprising (A) at least one saturated acyclic terpane which is a saturated acyclic terpane having 10 to 40 carbon atoms, (B) at least one persulfate salt, and (C) at least one silicate selected from the group consisting of alkali metal silicates, alkali metal metasilicates and ammonium silicate.

In another aspect, the presently claimed invention is directed to a method for bleaching hair comprising at least the steps of:
a) combining the bleach powder as described herein with an aqueous medium to form a bleaching medium,
b) applying the bleaching medium to hair,
c) allowing the bleaching medium to remain on the hair for at least 2 to 50 minutes
d) rinsing the bleaching medium from the hair and
e) optionally drying the bleached hair.

In yet another aspect, the presently claimed invention is directed to the use of at least one saturated acyclic terpane which is a saturated acyclic terpane having 10 to 40 carbon atoms for reducing dusting of a bleach powder comprising at least one persulfate salt and at least one silicate selected from the group consisting of alkali metal silicates, alkali metal metasilicates and ammonium silicate.

Other objects, advantages and applications of the presently claimed invention will become apparent to those skilled in the art from the following detailed description.

### Detailed description

The following detailed description is merely exemplary in nature and is not intended to limit the presently claimed invention or the application and uses of the presently claimed invention. Furthermore, there is no intention to be bound by any theory presented in the preceding technical field, background, summary or the following detailed description.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

Furthermore, the terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

The term "about", when used in relation to a weight ratio or a weight percentage, means that a specific value should be understood as meaning a range of ± 10 %. For example, the weight proportion of a compound of about 1 %, means a weight proportion ranging from 0.9 to 1.1 %. For example, the weight proportion of a compound of about 2.5 %, means a weight proportion ranging from 2.25 to 2.75 %.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "preferred embodiment" means that a particular feature, structure, or characteristic described in connection with the embodiment is included in at least one embodiment of the presently claimed invention. Thus, appearances of the phrases "in one embodiment" or "in a preferred embodiment" in various places throughout this specification are not necessarily all referring to the same embodiment but may refer to different embodiments of the presently claimed invention. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

Furthermore, the ranges defined throughout the specification include the end values as well, i.e., a range of 1 to 10 implies that both 1 and 10 are included in the range. For the avoidance of doubt, the applicant shall be entitled to any equivalents according to applicable law.

For the purposes of the presently claimed invention, `% by weight' or 'wt.% 'as used in the presently claimed invention is with respect to the total weight of the bleach powder. Further, the sum of wt.% of all the compounds, as described herein, in the respective components adds up to 100 wt.%.

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

The measurement techniques described hereinabove and herein are well known to a person skilled in the art and therefore do not limit the presently claimed invention.

By "user" or "customer" it is meant the person preparing the hair treatment composition. The user is for example a professional hair stylist working in a salon and is different from the subject on whose hair the composition is applied, or the user is identical to the person on whose hair the composition is applied. The mixing can take place in a bowl or within a bottle or even through a mixer placed between the stored individual compositions and the point at which it is dispensed to be used. While two component bleach powders are the most commonly found in the market, there are also others which use three or more components.

By "lift" (or "lift power") it is meant the amount of lightening caused by the bleaching of the natural hair pigment melanin by using the bleach powder of the presently claimed invention. The amount of lift provided by different hair treatment compositions can be compared by using a human natural dark hair sample (e.g., dark hair of an individual of Chinese descent) and measuring the change of colour achieved following application of the compositions. The change in colour can be measured using well known parameters such as L*a*b* values. A composition can be said to provide a higher lift than another composition, when the resulting L* value or the so-called dL* value (given by L*_{final} - L*ᵢₙᵢₜᵢₐₗ) measured for a given treated sample of dark hair is higher for that composition than for the other composition, using the same experimental conditions. The denomination Level 2 (herein used interchangeably with "demi-permanent" or "tone-on-tone") and Level 3 (herein used interchangeably with "permanent") are commonly used in the hair colour trade to differentiate compositions with medium and high lift.

The below materials are being used in the embodiments of the presently claimed invention. The presently claimed invention is described with non-limiting embodiments. The listed ingredients are the preferred features as well as other preferred but non-limiting features of the presently claimed invention.

The measurement techniques described hereinabove and herein are well known to a person skilled in the art and therefore do not limit the presently claimed invention.

In one aspect, the presently claimed invention is directed to a bleach powder comprising (A) at least one saturated acyclic terpane which is a saturated acyclic terpane having 10 to 40 carbon atoms, (B) at least one persulfate salt, and (C) at least one silicate selected from the group consisting of alkali metal silicates, alkali metal metasilicates and ammonium silicate.

In an embodiment, the at least one saturated acyclic terpane (A) is a monoterpane, a sesquiterpane, a diterpane, a sesterterpane, a triterpane or a tetraterpane. In another embodiment, the at least one saturated acyclic terpane (A) is selected from the group consisting of 2,6-dimethyl octane, 2,6,10,15,19-pentamethylleicosane, farnesane, phytane, squalane, isosqualane, neosqualane and lycopane. In yet another embodiment, the at least one saturated acyclic terpane (A) is selected from the group consisting of squalane, isosqualane and neosqualane, preferably the at least one saturated acyclic terpane (A) is squalane.

As used herein, "squalane" refers to an oil and is also referred to as 2,6,10,15,19,23-hexamethyl tetracosane. In one embodiment, the squalane is synthetic squalane which is obtained by chemical synthesis or semisynthetic squalane which is obtained from naturally occurring squalene by reduction. Squalene is present in deep sea fish, such as sharks, or olive oil, rice bran oil, wheat germ oil, sesame oil and cotton seed oil. Squalane can also be obtained from sugar cane.

In a preferred embodiment, the bleach powder comprises squalane derived from a bio-based farnesene or squalane, isosqualane and neosqualane derived from a bio-based farnesene, i.e., the squalane, isosqualane and neosqualane which is produced from bio-based farnesene by catalytic reaction, chemical reaction, thermal reaction, hydrogenation or any combination thereof.

As used herein, the term "bio-based farnesene" refers to farnesene which is biologically produced from microorganisms, in particular genetically modified microorganisms, by fermentation of renewable carbon sources such as sugar. As used herein, "farnesene" refers to α-farnesene, β-farnesene or a mixture thereof. α -farnesene refers to a compound having the following structure: 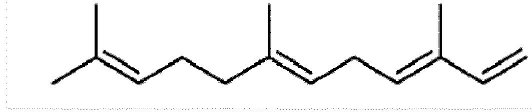 stereoisomer or a mixture of stereoisomers thereof.

β -farnesene refers to a compound having the following structure: 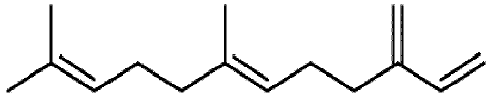 stereoisomer or a mixture thereof.

In one embodiment, β-farnesene is a substantially pure stereoisomer of β-farnesene. In another embodiment, β-farnesene comprises a mixture of stereoisomers, such as cistrans isomers. In another embodiment, the amount of each of the stereoisomers in the β-farnesene mixture is preferably in the range of ≥ 0.1 wt.% to ≤ 99.9 wt.%, more preferably in the range of ≥ 1.0 wt.% to ≤ 99.0 wt.%, even more preferably in the range of ≥ 5.0 wt.% to ≤ 95.0 wt.%, yet more preferably in the range of ≥ 10 wt.% to ≤ 90 wt.%, most preferably in the range of ≥ 20 wt.% to ≤ 80 wt.%, based on the total weight of the β-farnesene mixture.

In one embodiment, the at least one saturated acyclic terpane (A) has a degree of branching of 2, 3, 4, 5, 6 or 8, preferably the at least one saturated acyclic terpane (A) has a degree of branching of 6.

In yet another embodiment, the at least one component (A) is preferably present in an amount in the range of ≥ 0.5 wt.% to ≤ 15.0 wt.%, more preferably in the range of ≥ 1.0 wt.% to ≤ 14.0 wt.%, even more preferably in the range of ≥ 2.0 wt.% to ≤ 13.0 wt.%, yet even more preferably in the range of ≥ 2.0 wt.% to ≤ 10.0 wt.%, most preferably in the range of ≥ 2.0 wt.% to ≤ 8.0 wt.%, in particular in the range of ≥ 2.0 wt.% to ≤ 5.0 wt.%, based on the total weight of the bleach powder.

The bleach powder contains at least one persulfate salt (B). In one embodiment, the at least one persulfate salt (B) is selected from the group consisting of alkaline earth metal persulfate salts, alkali metal persulfate salts and ammonium persulfate salts that exhibit oxidizing activity, i.e., generating active oxygen, when combined with an aqueous composition comprising hydrogen peroxide. Examples of alkali metal persulfate salts include lithium persulfate, sodium persulfate and caesium persulfate. Examples of alkaline earth metal salts include magnesium persulfate and calcium persulfate. In one embodiment, the at least one persulfate salt (B) is preferably selected from the group consisting of ammonium persulfate, sodium persulfate and potassium persulfate. The persulfate salts are generally present in particulate form, having particle sizes in the range from ≥ 0.1 µm to ≤ 200 µm.

In one embodiment, the at least one component (B) is preferably present in an amount in the range of ≥ 20 wt.% to ≤ 70 wt.%, more preferably in the range of ≥ 25 wt.% to ≤ 65 wt.%, even more preferably in the range of ≥ 40 wt.% to ≤ 60 wt.%, based on the total weight of the bleach powder.

In one embodiment, the bleach powder comprises at least one silicate (C) which is sodium silicate. In another embodiment, the at least one component (C) is present in an amount in the range of ≥ 10 wt.% to ≤ 40 wt.%, preferably in the range of ≥ 15 wt.% to ≤ 35 wt.%, more preferably in the range of ≥ 20 wt.% to ≤ 32 wt.%, based on the total weight of the bleach powder. The sodium silicate is an alkaline salt which delivers an aqueous, basic pH, when the bleach powder according to the presently claimed invention is combined with an aqueous medium.

Since persulfate salts and hydrogen peroxide are relatively stable in acidic medium, it may be necessary to activate them at basic pH to obtain an adequate formulation. It is thus common practice to add to the bleach powder alkaline alkali metal silicates and/or ammonium silicates. The basic pH is an activating factor for enabling oxidation by an oxidizing agent such as hydrogen peroxide in combination with persulfate salts.

In one embodiment, the bleach powder according to the presently claimed invention preferably comprises at least one additive (D) selected from the group consisting of sources of carbonate ions or hydrogen carbonate ions or carbamate ions, aluminosilicates, anionic surfactants, chelants, thickening agents, fillers, amino acids, hydrolyzed proteins, C3-C20 monocarboxylic acids and C3-C10 di- or tricarboxylic acids. In another embodiment, the at least one additive (D) is preferably present in an amount in the range of ≥ 5.0 wt.% to ≤ 30 wt.%, more preferably in the range of ≥ 7.0 wt.% to ≤ 25 wt.%, even more preferably in the range of ≥ 10.0 wt.% to ≤ 22 wt.%, yet even more preferably in the range of ≥ 11 wt.% to ≤ 21 wt.%, most preferably in the range of ≥ 12 wt.% to ≤ 20 wt.%, in particular in the range of ≥ 13 wt.% to ≤ 20 wt.%, based on the total weight of the bleach powder.

In one embodiment, the sources of carbonate ions or hydrogen carbonate ions or carbamate ions are preferably selected from the group consisting of sodium, potassium, guanidine, arginine, lithium, calcium, magnesium, barium, ammonium salts of carbonate, carbamate and hydrogen carbonate ions. In another embodiment, the at least one source of carbonate ions or hydrogen carbonate ions or carbamate ions is preferably present in an amount in the range of ≥ 5.0 wt.% to ≤ 20 wt.%, more preferably in the range of ≥ 7.0 wt.% to ≤ 15 wt.%, even more preferably in the range of ≥ 8.0 wt.% to ≤ 12 wt.%, based on the total weight of the bleach powder. The sources of carbonate ions or hydrogen carbonate ions or carbamate ions are solid alkaline salts which deliver an aqueous, basic pH when the bleach powder is combined with an aqueous medium.

In one embodiment, the aluminosilicate is preferably sodium aluminosilicate. In another embodiment, the aluminosilicates are preferably present in an amount in the range of ≥ 10.0 wt.% to ≤ 35 wt.%, more preferably in the range of ≥ 12 wt.% to ≤ 30 wt.%, even more preferably in the range of ≥ 15 wt.% to ≤ 25 wt.%, based on the total weight of the bleach powder.

In one embodiment, the bleach powder according to the presently claimed invention preferably comprises anionic surfactants which are selected from the group consisting of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, alpha-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates; N-acyltaurates; and mixtures thereof. The alkyl or acyl radical of all of these various compounds, for example, comprises from 8 to 24 carbon atoms, and the aryl radical, for example, is chosen from phenyl and benzyl groups. Weakly anionic surfactants can also be used, such as alkyl-D-galactosiduronic acids and their salts, as well as polyoxyalkylenated (C6-C24) alkyl ether carboxylic acids, polyoxyalkylenated (C6-C24) alkylaryl ether carboxylic acids, polyoxyalkylenated (C6-C24) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, can be also used.

In one embodiment, the bleach powder according to the presently claimed invention comprises an anionic surfactants which is preferably selected from the group consisting of alkali metal salts and alkali earth metal salts of laurate, myristate, palmitate, stearate or behenate, more preferably the anionic surfactant is selected from the group consisting of sodium salts and potassium salts of laurate, palmitate or stearate. In one embodiment, the anionic surfactants are preferably present in an amount in the range of ≥ 2.0 wt.% to ≤ 9.0 wt.%, more preferably in the range of ≥ 3.0 wt.% to ≤ 8.0 wt.%, even more preferably in the range of ≥ 4.0 wt.% to ≤ 7.0 wt.%, based on the total weight of the bleach powder.

In one embodiment, the bleach powder preferably comprises additional surfactants selected from the group consisting of non-ionic surfactants, amphoteric or zwitterionic surfactants and cationic surfactants.

In one embodiment, nonionic surfactants are selected from the group consisting of fatty alcohol polyglycol ethers, alkylphenol polyglycol ethers, fatty acid polyglycol esters, fatty acid amide polyglycol ethers, fatty amine polyglycol ethers, alkoxylated triglycerides, optionally partly oxidized alk(en)yl oligoglycosides or glucuronic acid derivatives, fatty acid-N-alkyl glucamides, protein hydrolysates (particularly wheat-based vegetable products), polyol fatty acid esters, sugar esters, sorbitan esters, polysorbates and amine oxides.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate.

Amphoteric surfactants are surface-active compounds which, in addition to a C8 to C18 alkyl or acyl group, contain at least one free amino group and at least one -COOH or -SO₃H group in the molecule and which are capable of forming inner salts. Examples of suitable amphoteric surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride.

Hydrogen peroxide is easily decomposed when it comes into contact with heavy metals such as iron, copper and manganese and can quickly become ineffective. This drawback is usually eliminated by the incorporation of chelants into the formulation. In one embodiment, the bleach powder contains chelants which are preferably selected from the group consisting of ethylenediaminetetraacetic acid (EDTA), ethylenediamine-N,N'-disuccinic acid (EDDS), ethylenediamine-N,N'-diglutaric acid (EDDG), 2-hydroxypropyl-enediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid, diethylenetriamine pentaacetic acid (DTPA), ethylenedicysteic acid (EDC), N,N'-bis(2-hydroxybenzyl)ethylenediamine-N,N'-diacetic acid (HBED), ethylene diamine tri(methylene phosphonate), tetramethylethylenediamine (TMEDA), N,N,N',N'-tetraethylethylenediamine (TEEDA), and their salts thereof, more preferably the chelants are selected from the group consisting of ethylenediaminetetraacetic acid (EDTA), ethylenediamine-N,N'-disuccinic acid (EDDS), and their salts thereof. By "salts thereof", it is meant in the context of chelants all salts comprising the same functional structure as the chelant they are referring to and including alkali metal salts, alkaline earth salts, ammonium salts, substituted ammonium salts, and mixtures thereof; alternatively sodium salts, potassium salts, calcium salts, magnesium salts, ammonium salts, and mixtures thereof; alternatively monoethanolammonium salts, diethanolammonium salts, triethanolammonium salts, and mixtures thereof.

In one embodiment, the chelants are preferably present in an amount in the range of ≥ 0.4 wt.% to ≤ 3.0 wt.%, more preferably in the range of ≥ 0.5 wt.% to ≤ 2.0 wt.%, even more preferably in the range of ≥ 0.7 wt.% to ≤ 1.5 wt.%, based on the total weight of the bleach powder.

The bleach powder according to the presently claimed invention comprises preferably at least one thickening agent. Thickening agents are typically water-soluble and deliver the desired semi-fluid to pasty consistency to the bleaching medium. Typical thickening agents include, but are not limited to, synthetic water-soluble polymers and water-soluble polymers of natural origin. In one embodiment, the bleach powder according to the presently claimed invention comprises thickening agents which are preferably selected from the group consisting of cellulose derivatives, a polyacrylic acids, acrylate copolymers, polysaccharide gums, xanthan gums, starch, guars, starch derivatives, alginic acid and acrylic acid-diallyldimethylammonium polymers; more preferably are selected from the group consisting of xanthan gums, starch, and alginic acid.

Exemplary embodiments of water-soluble synthetic polymeric thickening agents comprise polyvinylpyrrolidone, polyacrylic acid, polyacrylamide, non-crosslinked poly-2-acrylamidopropanesulfonic acid, acid crosslinked poly-2-acrylamido-2-methylpropane sulfonic acid, cross-linked poly-2-acrylamido-2-methylpropane sulfonic acid partially neutralized by ammonia. Additional exemplary embodiments of synthetic water-soluble thickening agents include those such as non-crosslinked poly-2-acrylamido-2-methylpropane sulfonic acid in combination with hydroxyalkylcellulose ethers or with poly (ethylene oxide). Embodiments of water-soluble thickening agents derived from natural sources include, but are not limited to, polymers comprising at least one sugar unit. Exemplary embodiments of natural source thickening agents include by are not limited to non-ionic guar gums semisynthetic guar gums modified with C1-C6 hydroxylalkyl groups; biopolysaccharide gums of microbial origin such as scleroglucan or xanthan gums; gums from plant exudates such as arabian, ghatti, karaya, tragacanth, carrageenan, agar and carob gums; pectins; alginates; starches including such examples as rice starch, corn starch, potato starch, canola starch, cassava starch and hydrolyzed derivatives thereof; hydroxyalkyl (C 1 -C 6 ) celluloses and carboxyalkyl (C1 -C 6 ) celluloses. Embodiments of water-soluble cellulosic thickening agents include, but are not limited to, hydroxyalkyl (C 1 -C 6 ) celluloses such as hydroxyethylcellulose.

In one embodiment, the thickening agents are preferably present in an amount in the range of ≥ 2.0 wt.% to ≤ 10.0 wt.%, more preferably in the range of ≥ 3.5 wt.% to ≤ 8.0 wt.%, even more preferably in the range of ≥ 4.0 wt.% to ≤ 6.0 wt.%, based on the total weight of the bleach powder.

In one embodiment, the bleach powder according to the presently claimed invention comprises fillers which are selected from the group consisting of kaolin clay, smectite clay and a sepiolite clay. In one embodiment, the fillers are preferably present in an amount in the range of ≥ 2.0 wt.% to ≤ 10.0 wt.%, more preferably in the range of ≥ 3.5 wt.% to ≤ 8.0 wt.%, even more preferably in the range of ≥ 4.0 wt.% to ≤ 6.0 wt.%, based on the total weight of the bleach powder.

In one embodiment, the bleach powder preferably comprises at least one amino acid selected from the group consisting of alanine, glycine, phenylalanine, leucine, valine, isoleucine, glutamic acid, aspartic acid, citrulline, histidine, arginine, lysine and tyrosine; more preferably at least one amino acid selected from the group consisting of alanine, glycine, valine and arginine; most preferably the at least one amino acid is alanine or glycine or valine or arginine. In a preferred embodiment, the bleach powder does not contain other amino acids. The at least one amino acid which is present in the bleach powder of the presently claimed invention is oxidatively stable in a bleaching medium, i.e., the at least one amino acid is not oxidized or only oxidized to a very small degree in a bleaching medium by one of a nucleophilic oxidation reaction, an electrophilic oxidation reaction or a radical oxidation reaction. In one embodiment, the at least one amino acid is preferably obtained from a natural source, more preferably the at least one amino acid is obtained from a plant source. In one embodiment, the amino acids are preferably present in an amount in the range of ≥ 0.05 wt.% to ≤ 2.0 wt.%, more preferably in the range of ≥ 0.1 wt.% to ≤ 1.5 wt.%, even more preferably in the range of ≥ 0.1 wt.% to ≤ 1.3 wt.%, yet even more preferably in the range of ≥ 0.1 wt.% to ≤ 1.0 wt.%, based on the total weight of the bleach powder.

In one embodiment, the bleach powder preferably comprises hydrolyzed proteins which are selected from the group consisting of hydrolyzed keratin and hydrolyzed vegetable protein. Hydrolyzed proteins are commercially available in the market, e.g., AC Vegan Keratin OS, Nutrilan^{®} Keratin LM, Gluadin^{®} Kera-P LM, KeraMatch^{®} V, FK Repair Ultra, FK Restore, FK Protect Plus and ProSina^{™}.

In one embodiment, the bleach powder according to the presently claimed invention comprises C3-C20 monocarboxylic acids which are preferably selected from the group consisting of propionic acid, butyric acid, pentanoic acid, iso-pentanoic acid, hexanoic acid, iso-hexanoic acid, heptanoic acid, iso-heptanoic acid, octanoic acid, iso-octanoic acid, nonanoic acid, iso-nonanoic acid, decanoic acid, undecanoic acid, dodecanoic acid, tridecanoic acid, tetradecanoic acid, pentadecanoic acid, palmitic acid, heptadecanoic acid, stearic acid, oleic acid, linoleic acid, linolenic acid, nonadecanoic acid and eicosanoic acid. In one embodiment, the C3-C20 monocarboxylic acids are preferably present in an amount in the range of ≥ 0.05 wt.% to ≤ 2.0 wt.%, more preferably in the range of ≥ 0.1 wt.% to ≤ 1.5 wt. %, even more preferably in the range of ≥ 0.1 wt.% to ≤ 1.3 wt. %, yet even more preferably in the range of ≥ 0.1 wt.% to ≤ 1.0 wt. %, based on the total weight of the bleach powder.

In one embodiment, the bleach powder according to the presently claimed invention comprises C3-C10 di- or tricarboxylic acids which are preferably selected from the group consisting of tartronic acid, succinic acid, malic acid, maleic acid, citric acid, tartaric acid, adipic acid, glutaric acid, oxalic acid, sebacic acid, glyoxylic acid, malonic acid and hydroxyglutaric acid. In one embodiment, the C3-C10 di- or tricarboxylic acids are preferably present in an amount in the range of ≥ 0.05 wt.% to ≤ 2.0 wt.%, more preferably in the range of ≥ 0.1 wt.% to ≤ 1.5 wt.%, even more preferably in the range of ≥ 0.1 wt.% to ≤ 1.3 wt.%, yet even more preferably in the range of ≥ 0.1 wt.% to ≤ 1.0 wt.%, based on the total weight of the bleach powder.

In one embodiment, the bleach powder according to the presently claimed invention comprises
(A) ≥ 2.0 wt.% to ≤ 5.0 wt.% of squalane,
(B) ≥ 40 wt.% to ≤ 60 wt.% of at least one persulfate salt,
(C) ≥ 20 wt.% to ≤ 32 wt.% of sodium silicate, and
(D) ≥ 12 wt.% to ≤ 25 wt.% of at least one additive (D) selected from the group consisting of sources of carbonate ions or hydrogen carbonate ions or carbamate ions, aluminosilicates, anionic surfactants, chelants, thickening agents, fillers, amino acids, hydrolyzed proteins, C3-C20 monocarboxylic acids and C3-C10 di- or tricarboxylic acids.

In one embodiment, the bleach powder of the presently claimed invention is preferably prepared by dispersing, under mechanical action, all the compounds that are present in particle form, i.e., the at least one persulfate salt (B) and the at least one silicate (C), in the at least one saturated acyclic terpane which is a saturated acyclic terpane having 10 to 40 carbon atoms (A), in which the other liquid components of the bleach powder have been pre-dispersed or premixed.

In another embodiment, the bleach powder of the presently claimed invention is preferably prepared by extrusion, by introducing the liquid and solid phases of the bleach powder into the extruder, and then mixing all components at a temperature ≤ 25 °C, using a co-rotating twin-screw system comprising transportation and blending means.

The bleach powder according to the presently claimed invention is used in combination with an oxygen donor, such as hydrogen peroxide, to bleach hair. Thus, in another aspect the presently claimed invention is directed to a method for bleaching hair comprising at least the steps of:
a) combining the bleach powder as describe herein with an aqueous medium to form a bleaching medium,
b) applying the bleaching medium to hair, which is dry or wet,
c) allowing the bleaching medium to remain on the hair for at least 2 to 50 minutes
d) rinsing the bleaching medium from the hair and
e) optionally drying the bleached hair.

The bleaching medium is preferably applied to the air with any number of application devices, with the main purpose of scarcely and densely depositing the bleach medium on the hair to achieve the desired bleaching result.

In one embodiment, the aqueous medium preferably contains hydrogen peroxide. In step a), the bleach powder is preferably mixed with 0.5 to 10 weight equivalents of the aqueous medium which may be a solution, an emulsion or a gel, containing ≥ 2.0 wt.% to ≤ 12.0 wt.% of hydrogen peroxide, based on the total weight of the aqueous medium. The combination is preferably performed immediately before applying the bleaching medium to the hair.

In one embodiment, the aqueous medium has a pH of ≤ 7.0. The acidic pH ensures the stability of the hydrogen peroxide in the aqueous medium. The pH is preferably obtained by the addition of at least one acidifying agent selected from the group consisting of hydrochloric acid, acetic acid, phosphoric acid, lactic acid and boric acid. The aqueous medium preferably comprises at least one adjuvant selected from the group consisting of preserving agents, colorants, fragrances, anti-foaming agents, hydrogen peroxide stabilizers, and chelants such as EDTA and EDDS.

In one aspect, the presently claimed invention is directed to the use of at least one saturated acyclic terpane which is a saturated acyclic terpane having 10 to 40 carbon atoms for reducing dusting of a bleach powder comprising at least one persulfate salt and at least one silicate selected from the group consisting of alkali metal silicates, alkali metal metasilicates and ammonium silicate. In one embodiment, the saturated acyclic terpane having 10 to 40 carbon atoms is preferably squalane.

Oxidative hair colouring compositions are typically sold in the form of kits comprising individually packaged components such as separate containers, e.g., a dye or tint component comprising a dye coupler and precursors; an aqueous hydrogen peroxide component and a bleach powder comprising the alkalizing agent and the persulfate salts. Hence, in another aspect, the presently claimed invention is also directed to a kit comprising i) an individually packaged oxidizing component comprising hydrogen peroxide; ii) an individually packaged colouring component comprising at least one dye coupler and at least one dye precursor; and iii) an individually packaged bleach powder as described herein.

In one embodiment, the dye precursors are selected from of the group consisting of toluene-2,5-diamine, p-phenylenediamine, n-phenyl-p-phenylenediamine, N,N-bis(2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof. Preferably, the oxidative primary dye precursors are selected from of the group consisting of toluene-2,5-diamine, 4,5-diamino-1-hexylpyrazol, N,N-bis(2-hydroxyethyl)-p-phenylenediamine, hydroxypropyl-p-phenylenediamine, 2-methoxymethyl-p-phenylenediamine and salts thereof.

In one embodiment, the dye couplers are selected from the group consisting of resorcinol, 4-chlororesorcinol, 2-chlororesorcinol, 2-methylresorcinol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzomorpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotolueneµ, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindole, 6-hydroxyindole, dihydroxyindoline, phenyl methyl pyrazolone, 1,2,4-trihydroxybenzene, 5-((2-hydroxyethyl)amino)-1,3-benzodioxol, isatin, hydroquinone, 4-formyl-1-methylquinolinium-p-toluenesulfonate and salts thereof. Preferably, the oxidative coupler dye precursors are selected from the group consisting of resorcinol, 2-methylresorcinol, 4-chlororesorcinol, 2-chlororesorcinol, m-aminophenol, hydroxybenzomorpholine, 2,4-diaminophenoxyethanol, 4-amino-2-hydroxytoluene, 2-methyl-5-hydroxyethylaminophenol, 5-((2-hydroxyethyl)amino)-1,3-benzodioxol and salts thereof.

The presently claimed invention is associated with one or more of the following advantages.
- The bleach powder shows little dusting upon application by hair stylists.
- The bleach powder is mixed into a hair bleaching medium that is stable and does not show any phase separation or only little phase separation. The hair bleaching medium allows for better application on a customer's hair.
- The bleach powder is formulated into a hair bleaching medium that effectively colours hair and effectively covers grey hair.
- The bleach powder is formulated into a hair bleaching medium that reduces the level of damage of hair that occurs during the hair bleaching process.

In the following, there is provided a list of embodiments to further illustrate the present disclosure without intending to limit the disclosure to the specific embodiments listed below.

### Embodiments

Embodiment 1. A bleach powder comprising
(A) at least one saturated acyclic terpane which is a saturated acyclic terpane having 10 to 40 carbon atoms,
(B) at least one persulfate salt, and
(C) at least one silicate selected from the group consisting of alkali metal silicates, alkali metal metasilicates and ammonium silicate.

Embodiment 2. The bleach powder according to embodiment 1, wherein the at least one saturated acyclic terpane (A) is a monoterpane, a sesquiterpane, a diterpane, a sesterterpane, a triterpane or a tetraterpane.

Embodiment 3. The bleach powder according to embodiment 1, wherein the at least one saturated acyclic terpane (A) is selected from the group consisting of 2,6-dimethyl octane, 2,6,10,15,19-pentamethylleicosane, farnesane, phytane, squalane, isosqualane, neosqualane and lycopane.

Embodiment 4. The bleach powder according to embodiment 3, wherein the at least one saturated acyclic terpane (A) is selected from the group consisting of squalane, isosqualane and neosqualane.

Embodiment 5. The bleach powder according to embodiment 1, wherein the at least one saturated acyclic terpane (A) has a degree of branching of 2, 3, 4, 5, 6 or 8.

Embodiment 6. The bleach powder according to embodiment 5, wherein the at least one saturated acyclic terpane (A) has a degree of branching of 6.

Embodiment 7. The bleach powder according to any of the preceding embodiments, wherein the at least one saturated acyclic terpane (A) is derived from a bio-based compound.

Embodiment 8. The bleach powder according to embodiment 7, wherein the bio-based compound is selected from the group consisting of squalene and farnesene.

Embodiment 9. The bleach powder according to any of the preceding embodiments, wherein the at least one component (A) is present in an amount in the range of ≥ 0.5 wt.% to ≤ 15.0 wt.%, preferably in the range of ≥ 1.0 wt.% to ≤ 14.0 wt.%, more preferably in the range of ≥ 2.0 wt.% to ≤ 13.0 wt.%, even more preferably in the range of ≥ 2.0 wt.% to ≤ 10.0 wt.%, most preferably in the range of ≥ 2.0 wt. % to ≤ 8.0 wt. %, in particular in the range of ≥ 2.0 wt. % to ≤ 5.0 wt. %, based on the total weight of the bleach powder.

Embodiment 10. The bleach powder according to any of the preceding embodiments, wherein the at least one persulfate salt (B) is selected from the group consisting of ammonium persulfate, sodium persulfate and potassium persulfate.

Embodiment 11. The bleach powder according to any of the preceding embodiments, wherein the at least one component (B) is present in an amount in the range of ≥ 20 wt.% to ≤ 70 wt.%, preferably in the range of ≥ 25 wt.% to ≤ 65 wt.%, more preferably in the range of ≥ 40 wt.% to ≤ 60 wt.%, based on the total weight of the bleach powder.

Embodiment 12. The bleach powder according to any of the preceding embodiments, wherein the at least one silicate (C) is sodium silicate.

Embodiment 13. The bleach powder according to any of the preceding embodiments, wherein the at least one component (C) is present in an amount in the range of ≥ 10 wt.% to ≤ 40 wt.%, preferably in the range of ≥ 15 wt.% to ≤ 35 wt.%, more preferably in the range of ≥ 20 wt.% to ≤ 32 wt.%, based on the total weight of the bleach powder.

Embodiment 14. The bleach powder according to any of the preceding embodiments, wherein the bleach powder comprises at least one additive (D) selected from the group consisting of sources of carbonate ions or hydrogen carbonate ions or carbamate ions, aluminosilicates, anionic surfactants, chelants, thickening agents, fillers, amino acids, hydrolyzed proteins, C3-C20 monocarboxylic acids and C3-C10 di- or tricarboxylic acids.

Embodiment 15. The bleach powder according to embodiment 14, wherein the at least one additive (D) is present in an amount in the range of ≥ 5.0 wt.% to ≤ 30 wt.%, preferably in the range of ≥ 7.0 wt.% to ≤ 25 wt.%, more preferably in the range of ≥ 10.0 wt.% to ≤ 22 wt.%, even more preferably in the range of ≥ 11 wt.% to ≤ 21 wt.%, most preferably in the range of ≥ 12 wt.% to ≤ 20 wt.%, in particular in the range of ≥ 13 wt.% to ≤ 20 wt.%, based on the total weight of the bleach powder.

Embodiment 16. The bleach powder according to embodiment 14, wherein the sources of carbonate ions or hydrogen carbonate ions or carbamate ions is selected from the group consisting of sodium, potassium, guanidine, arginine, lithium, calcium, magnesium, barium, ammonium salts of carbonate, carbamate and hydrogen carbonate ions.

Embodiment 17. The bleach powder according to any one of embodiments 14 to 16, wherein the at least one source of carbonate ions or hydrogen carbonate ions or carbamate ions is present in an amount in the range of ≥ 5.0 wt.% to ≤ 20 wt.%, preferably in the range of ≥ 7.0 wt.% to ≤ 15 wt.%, more preferably in the range of ≥ 8.0 wt.% to ≤ 12 wt.%, based on the total weight of the bleach powder.

Embodiment 18. The bleach powder according to embodiment 14, wherein the aluminosilicate is sodium aluminosilicate.

Embodiment 19. The bleach powder according to any one of embodiments 14 to 18, wherein the aluminosilicates are present in an amount in the range of ≥ 10.0 wt.% to ≤ 35 wt.%, preferably in the range of ≥ 12 wt.% to ≤ 30 wt.%, more preferably in the range of ≥ 15 wt.% to ≤ 25 wt.%, based on the total weight of the bleach powder.

Embodiment 20. The bleach powder according to any one of embodiments 14 to 19, wherein the anionic surfactants are selected from the group consisting of alkali metal salts and alkali earth metal salts of laurate, myristate, palmitate, stearate or behenate.

Embodiment 21. The bleach powder according to any one of embodiments 14 to 19, wherein the anionic surfactant is selected from the group consisting of sodium salts and potassium salts of laurate, palmitate or stearate.

Embodiment 22. The bleach powder according to any one of embodiments 14 to 20, wherein the anionic surfactants are present in an amount in the range of ≥ 2.0 wt.% to ≤ 9.0 wt.%, preferably in the range of ≥ 3.0 wt.% to ≤ 8.0 wt.%, more preferably in the range of ≥ 4.0 wt.% to ≤ 7.0 wt.%, based on the total weight of the bleach powder.

Embodiment 22. The bleach powder according to any one of embodiments 14 to 21, wherein the chelants are selected from the group consisting of ethylenediaminetetraacetic acid (EDTA), ethylenediamine-N,N'-disuccinic acid (EDDS), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid, diethylenetriamine pentaacetic acid (DTPA), ethylenedicysteic acid (EDC), N,N'-bis(2-hydroxybenzyl)ethylenediamine-N,N'-diacetic acid (HBED), ethylene diamine tri(methylene phosphonate), tetramethylethylenediamine (TMEDA), N,N,N',N'-tetraethylethylenediamine (TEEDA), and their salts thereof.

Embodiment 24. The bleach powder according to embodiment 23, wherein the chelants are selected from the group consisting of ethylenediaminetetraacetic acid (EDTA), ethylenediamine-N,N'-disuccinic acid (EDDS), and their salts thereof.

Embodiment 25. The bleach powder according to any one of embodiments 14 to 24, wherein the chelants are present in an amount in the range of ≥ 0.4 wt.% to ≤ 3.0 wt.%, preferably in the range of ≥ 0.5 wt.% to ≤ 2.0 wt.%, more preferably in the range of ≥ 0.7 wt.% to ≤ 1.5 wt.%, based on the total weight of the bleach powder.

Embodiment 26. The bleach powder according to any one of embodiments 14 to 25, wherein the thickening agents are selected from the group consisting of cellulose derivatives, a polyacrylic acids, acrylate copolymers, polysaccharide gums, xanthan gums, starch, guars, starch derivatives, alginic acid and acrylic acid-diallyldimethylammonium polymers.

Embodiment 27. The bleach powder according to embodiment 26, wherein the thickening agents are selected from the group consisting of xanthan gums, starch, and alginic acid.

Embodiment 28. The bleach powder according to any one of embodiments 14 to 27, wherein the thickening agents are present in an amount in the range of ≥ 2.0 wt.% to ≤ 10.0 wt.%, preferably in the range of ≥ 3.5 wt.% to ≤ 8.0 wt.%, more preferably in the range of ≥ 4.0 wt.% to ≤ 6.0 wt.%, based on the total weight of the bleach powder.

Embodiment 29. The bleach powder according to any one of embodiments 14 to 28, wherein the fillers are selected from the group consisting of kaolin clay, smectite clay and a sepiolite clay.

Embodiment 30. The bleach powder according to any one of embodiments 14 to 29, wherein the fillers are present in an amount in the range of ≥ 2.0 wt.% to ≤ 10.0 wt.%, preferably in the range of ≥ 3.5 wt.% to ≤ 8.0 wt.%, more preferably in the range of ≥ 4.0 wt.% to ≤ 6.0 wt.%, based on the total weight of the bleach powder.

Embodiment 31. The bleach powder according to any one of embodiments 14 to 30, wherein the amino acids are selected from the group consisting of alanine, glycine, phenylalanine, leucine, valine, isoleucine, glutamic acid, aspartic acid, citrulline, histidine, arginine, lysine and tyrosine.

Embodiment 32. The bleach powder according to any one of embodiments 14 to 31, wherein the amino acids are present in an amount in the range of ≥ 0.05 wt.% to ≤ 2.0 wt.%, preferably in the range of ≥ 0.1 wt.% to ≤ 1.5 wt.%, more preferably in the range of ≥ 0.1 wt.% to ≤ 1.3 wt.%, even more preferably in the range of ≥ 0.1 wt.% to ≤ 1.0 wt.%, based on the total weight of the bleach powder.

Embodiment 33. The bleach powder according to any one of embodiments 14 to 32, wherein the hydrolyzed proteins are selected from the group consisting of hydrolyzed keratin and hydrolyzed vegetable protein.

Embodiment 34. The bleach powder according to any one of embodiments 14 to 33, wherein the C3-C10 di- or tricarboxylic acids are selected from the group consisting of tartronic acid, succinic acid, malic acid, maleic acid, citric acid, tartaric acid, adipic acid, glutaric acid, oxalic acid, sebacic acid, glyoxylic acid, malonic acid and hydroxyglutaric acid.

Embodiment 35. The bleach powder according to any one of embodiments 14 to 34, wherein the C3-C20 monocarboxylic acids or C3-C10 di- or tricarboxylic acids are each present in an amount in the range of ≥ 0.05 wt.% to ≤ 2.0 wt.%, preferably in the range of ≥ 0.1 wt.% to ≤ 1.5 wt.%, more preferably in the range of ≥ 0.1 wt.% to ≤ 1.3 wt.%, even more preferably in the range of ≥ 0.1 wt.% to ≤ 1.0 wt.%, based on the total weight of the bleach powder.

Embodiment 36. The bleach powder according to any one of embodiments 1 to 35 comprising
(A) ≥ 2.0 wt.% to ≤ 5.0 wt.% of squalane,
(B) ≥ 40 wt.% to ≤ 60 wt.% of at least one persulfate salt,
(C) ≥ 20 wt.% to ≤ 32 wt.% of sodium silicate, and
(D) ≥ 12 wt.% to ≤ 25 wt.% of at least one additive (D) selected from the group consisting of sources of carbonate ions or hydrogen carbonate ions or carbamate ions, aluminosilicates, anionic surfactants, chelants, thickening agents, fillers, amino acids, hydrolyzed proteins, C3-C20 monocarboxylic acids and C3-C10 di- or tricarboxylic acids.

Embodiment 37. A method for bleaching hair comprising at least the steps of:
a) combining the bleach powder according to any one of embodiments 1 to 36 with an aqueous medium to form a bleaching medium,
b) applying the bleaching medium to hair,
c) allowing the bleaching medium to remain on the hair for at least 2 to 50 minutes
d) rinsing the bleaching medium from the hair and
e) optionally drying the bleached hair.

Embodiment 38. Use of at least one saturated acyclic terpane which is a saturated acyclic terpane having 10 to 40 carbon atoms for reducing dusting of a bleach powder comprising at least one persulfate salt and at least one silicate selected from the group consisting of alkali metal silicates, alkali metal metasilicates and ammonium silicate.

Embodiment 39. The use according to embodiment 38, wherein the saturated acyclic terpane having 10 to 40 carbon atoms is squalane.

While the presently claimed invention has been described in terms of its specific embodiments, certain modifications and equivalents will be apparent to those skilled in the art and are intended to be included within the scope of the presently claimed invention.

### Examples

The presently claimed invention is illustrated in detail by non-restrictive working examples which follow. More particularly, the test methods specified hereinafter are part of the general disclosure of the application and are not restricted to the specific working examples.

### Materials

- Potassium persulfate commercially available from United Initiators, Pullach, Germany
- Alkaline sodium silicate commercially available from BASF, Ludwigshafen/Rhine, Germany
- Ammonium persulfate commercially available from United Initiators, Pullach, Germany
- Magnesium carbonate hydroxide commercially available from Dr. Paul Lohmann, Emmerthal, Germany
- Sodium stearate commercially available from Matrix Chemie
- Squalane commercially available from Aprinnova
- Xanthan gum commercially available from KP Kelco, Großenbrode, Germany
- Algin commercially available from BASF, Ludwigshafen/Rhine, Germany
- Disodium EDTA commercially available from BASF, Ludwigshafen/Rhine, Germany
- D,L-Malic acid commercially available from Merck Chemicals GmbH, Darmstadt, Germany
- Mineral oil commercially available from Panama PetroChem Ltd., India

### Test Methods

### Combing Force Measurement

Various external influences, such as certain cosmetic treatments (bleaching, colouring, permanent waving), exposure to the weather, frequent combing and brushing affect the combing ease due to a degradation of the hair cuticle. Thus, the combing ease of hair is a major parameter for describing the quality/degree of damage of hair on the one hand and/or the effectiveness of conditioning respectively hair repair treatments on the other hand. The principle of most methods for the determination of combing properties consists in measuring the force to pull a comb through a hair tress under definite conditions (Ref.: C.R. Robbins, Chemical and Physical Behavior of Human Hair, 5th Edition, p. 646 ff., Springer-Verlag, Berlin Heidelberg 2012. ISBN 978-3-642-25610-3). For this test, an automated device was used in which the tresses to be tested are taken from a storage chamber and are fixed to a force meter. The tresses were combed automatically at constant speed. For each combing stroke the combing force was recorded as a function of the distance combed through (length of the tress). For comparison purposes 20 combing strokes per tress on at least 3 tresses per product sample were averaged. The Wet Combing Force was the average force along the tress. The lower the combing force the better the combing ease.

### Formulations

The samples were prepared by mixing all the dry ingredients together for 5 minutes inside a mixer where the rotational speed of the mixing blade was 3.5 rpm. Dedusting component was in a drop by drop manner while the rotational speed was set at 3 rpm and finally the speed was increased to 3.5 rpm for 6 minutes which ensured a homogenous mixture.

**Table 1.**

| | **Sample 1 in wt.-%** | **Sample 2* in wt.-%** |
|---|---|---|
| Potassium persulfate | 44,0 | 44,0 |
| Alkaline sodium silicate | 26,0 | 26,0 |
| Ammonium persulfate | 8,0 | 8,0 |
| Magnesium carbonate hydroxide | 8,8 | 8,8 |
| Sodium stearate | 4,0 | 4,0 |
| Squalane | 3,2 | 0 |
| Xanthan gum | 2,8 | 2,8 |
| Algin | 1,0 | 1,0 |
| Disodium EDTA | 1,3 | 1,3 |
| D,L-Malic acid | 0,9 | 0,9 |
| Mineral oil | 0 | 3,2 |

| | | |
|---|---|---|
| * not within the scope of the presently claimed invention | | |

### Example 1 and 2: Combing tests

In the examples of the presently claimed invention either Welloxon^{®} Perfect 6%, or 9% oxidizing formula containing 6 wt.% or 9 wt.% hydrogen peroxide in an aqueous medium, based on the overall weight of the product (Developer from the Wella Germany GmbH, Darmstadt, Germany) was used.

Bleaching compositions produced by mixing bleach powders (Sample 1 and 2) described in Table 1 with a market developer, as described above, were tested for their impact on hair combability and smoothness.

The following protocol was used to assess the performance of each oxidative hair treatment composition.
1. Three natural Caucasian hair tresses (Kerling) were used
2. Apply a bleaching composition prepared using Welloxon^{®} Perfect 9% and Blondor^{®}.
3. 1 part of each bleach powder was mixed with 1,5 parts of oxidizing composition (Welloxon^{®} Perfect 9%) and then applied and worked throughout the hair tresses with a brush. 4 g of product was applied to each 1 g tress.
3. The tresses were placed into a 30 °C oven for 30 minutes for the bleaching process to occur.
4. The tresses were then removed from the oven and rinsed in water, 4 L min⁻¹ at a temperature of 37 +- 2 °C for 2 minutes
5. The tresses are combed twice (rough & fine side of comb) and placed inside between pre-wettened paper sheets.
6. Combing Force measurements were performed 3 times for each tress.

The measurement results are presented in Table 2.

**Table 2: Combing Force measurements**

| Example | Sample | Combing Force Average, N | Combing Force Standard deviation, N |
|---|---|---|---|
| 1 | 1 | 0,276 | 0,037 |
| 2* | 2* | 0,305 | 0,020 |

| | | | |
|---|---|---|---|
| * not within the scope of the presently claimed invention | | | |

Results show that tresses treated with bleach powder based on sample 1 containing squalane mixed with Welloxon^{®} Perfect 9% required statistically significantly less force to comb through than the ones treated with the bleach powder containing mineral oil. It showed that the addition of squalane reduced mechanical damage when combing hair.

### Example 3 and 4: Dedusting

The bleach powder samples 1 and 2 were mixed with Welloxon^{®} developer. While pouring the powder product from the container, prior to mixing, 3 stylists assessed the prototypes 10 times and reported less dusting or similar dusting behavior to the reference dedusted with mineral oil.

## Claims

1. A bleach powder comprising
(A) at least one saturated acyclic terpane which is a saturated acyclic terpane having 10 to 40 carbon atoms,
(B) at least one persulfate salt, and
(C) at least one silicate selected from the group consisting of alkali metal silicates, alkali metal metasilicates and ammonium silicate.

2. The bleach powder according to claim 1, wherein the at least one saturated acyclic terpane (A) is selected from the group consisting of squalane, isosqualane and neosqualane.

3. The bleach powder according to any of the preceding claims, wherein the at least one component (A) is present in an amount in the range of ≥ 0.5 wt.% to ≤ 15.0 wt.%, preferably in the range of ≥ 1.0 wt.% to ≤ 14.0 wt.%, more preferably in the range of ≥ 2.0 wt.% to ≤ 13.0 wt.%, even more preferably in the range of ≥ 2.0 wt.% to ≤ 10.0 wt.%, most preferably in the range of ≥ 2.0 wt.% to ≤ 8.0 wt.%, in particular in the range of ≥ 2.0 wt.% to ≤ 5.0 wt.%, based on the total weight of the bleach powder.

4. The bleach powder according to any of the preceding claims, wherein the at least one persulfate salt (B) is selected from the group consisting of ammonium persulfate, sodium persulfate and potassium persulfate.

5. The bleach powder according to any of the preceding claims, wherein the at least one component (B) is present in an amount in the range of ≥ 20 wt.% to ≤ 70 wt.%, preferably in the range of ≥ 25 wt.% to ≤ 65 wt.%, more preferably in the range of ≥ 40 wt.% to ≤ 65 wt.%, based on the total weight of the bleach powder.

6. The bleach powder according to any of the preceding claims, wherein the at least one silicate (C) is sodium silicate.

7. The bleach powder according to any of the preceding claims, wherein the at least one component (C) is present in an amount in the range of ≥ 10 wt.% to ≤ 40 wt.%, preferably in the range of ≥ 15 wt.% to ≤ 35 wt.%, more preferably in the range of ≥ 20 wt.% to ≤ 32 wt.%, based on the total weight of the bleach powder.

8. The bleach powder according to any of the preceding claims, wherein the bleach powder comprises at least one additive (D) selected from the group consisting of sources of carbonate ions or hydrogen carbonate ions or carbamate ions, aluminosilicates, anionic surfactants, chelants, thickening agents, fillers, amino acids, hydrolyzed proteins, C3-C20 monocarboxylic acids and C3-C10 di- or tricarboxylic acids.

9. The bleach powder according to claim 8, wherein the at least one additive (D) is present in an amount in the range of ≥ 5.0 wt.% to ≤ 30 wt.%, preferably in the range of ≥ 7.0 wt.% to ≤ 25 wt.%, more preferably in the range of ≥ 10.0 wt.% to ≤ 22 wt.%, even more preferably in the range of ≥ 11 wt.% to ≤ 21 wt.%, most preferably in the range of ≥ 12 wt.% to ≤ 20 wt.%, in particular in the range of ≥ 13 wt.% to ≤ 20 wt.%, based on the total weight of the bleach powder.

10. The bleach powder according to any of the preceding claims comprising
(A) ≥ 2.0 wt.% to ≤ 5.0 wt.% of squalane,
(B) ≥ 40 wt.% to ≤ 60 wt.% of at least one persulfate salt,
(C) ≥ 20 wt.% to ≤ 32 wt.% of sodium silicate, and
(D) ≥ 12 wt.% to ≤ 25 wt.% of at least one additive (D) selected from the group consisting of sources of carbonate ions or hydrogen carbonate ions or carbamate ions, aluminosilicates, anionic surfactants, chelants, thickening agents, fillers, amino acids, hydrolyzed proteins, C3-C20 monocarboxylic acids and C3-C10 di- or tricarboxylic acids.

11. A method for bleaching hair comprising at least the steps of:
a) combining the bleach powder according to any one of claims 1 to 10 with an aqueous medium to form a bleaching medium,
b) applying the bleaching medium to hair,
c) allowing the bleaching medium to remain on the hair for at least 2 to 50 minutes
d) rinsing the bleaching medium from the hair and
e) optionally drying the bleached hair.

12. Use of at least one saturated acyclic terpane which is a saturated acyclic terpane having 10 to 40 carbon atoms for reducing dusting of a bleach powder comprising at least one persulfate salt and at least one silicate selected from the group consisting of alkali metal silicates, alkali metal metasilicates and ammonium silicate.

13. The use according to claim 12, wherein the saturated acyclic terpane having 10 to 40 carbon atoms is squalane.
